# EUROPÄISCHE PATENTSCHRIFT

(11) **EP 1 404 725 B1**
(45) Veröffentlichungstag und Bekanntmachung des Hinweises auf die Patenterteilung: **16.11.2005**
(21) Anmeldenummer: 02758203.0
(22) Anmeldetag: 24.05.2002
(51) Int. Cl.: C08F 8/00

(54) **KOLLOIDKATALYSIERTE GASÜBERTRAGUNG IN ÜBERKRITISCHER PHASE**
COLLOID-CATALYSED GAS TRANSFER IN SUPERCRITICAL PHASES
TRANSPORT DE GAZ A CATALYSE COLLOIDALE EN PHASE SURCRITIQUE

(30) Priorität: 25.05.2001 DE 10125613
(43) Veröffentlichungstag der Anmeldung: 07.04.2004
(73) Patentinhaber: BASF Aktiengesellschaft, 67056 Ludwigshafen (DE)
(72) Erfinder: NIESSEN, Heiko, G., 50389 Wesseling (DE); WOELK, Klaus, 53343 Wachtberg (DE); EICHHORN, Andreas, 53347 Alfter (DE); BARGON, Joachim, 53177 Bonn (DE)
(74) Vertreter: Isenbruck, Günter
(86) Internationale Anmeldenummer: PCT/EP2002/005719
(87) Internationale Veröffentlichungsnummer: WO 2002/098925

(56) Entgegenhaltungen:
- EP-A- 0 841 314
- EP-A- 1 028 143
- WO-A-96/01304
- DE-A- 19 913 395
- US-A- 5 238 607
- M. V. SEREGINA: "PREPARATION OF NOBLE-METAL COLLOIDS IN BLOCK COPOLYMER MICELLES AND THEIR CATALYTIC PROPERTIES IN HYDROGENATION." AMERICAN CHEMICAL SOCIETY, Bd. 9, April 1997 (1997-04), Seiten 923-931, XP000659230
- L. M. BRONSTEIN: "THE CATALYTIC BEHAVIOR OF PT- AND RH-CONTAINING POLYMERS DERIVED FROM POLYSTYRENE-POLYBUTADIENE BLOCK COPOLYMERS IN HYDROSILYLATION." POLYMER BULLETIN, Bd. 40, Februar 1998 (1998-02), Seiten 173-180, XP000739858

## Beschreibung

Die Erfindung betrifft ein Verfahren zur Wasserstoffübertragung auf chemische Verbindungen durch Katalyse mit stabilisierten Metallkolloiden, sowie die Verwendung von stabilisierten Metallkolloiden in einem Verfahren zur Wasserstoffübertragung auf chemische Verbindungen.

Die Katalyse mit Metallkolloiden wird üblicherweise in konventionellen Lösungsmitteln durchgeführt.

DE-A 197 45 904 betrifft Metallkolloid-Lösungen, die durch mindestens ein Kationenaustauscherpolymer stabilisiert sind. Gemäß der Beschreibung sind die stabilisierten Metallkolloide in Wasser oder einem organischen Lösungsmittel löslich. Die in DE-A 197 45 904 offenbarten Metallkolloide sind als Katalysatoren, insbesondere für Brennstoffzellen, geeignet.

RU-C 2144020 betrifft ein Verfahren zur Hydrierung acetylenischer Alkohole zu den entsprechenden ethylenischen Alkoholen mit Wasserstoff unter Verwendung von micellaren, Palladium enthaltenden Katalysatoren. Die Katalysatoren sind aus Pd(0) aufgebaut, das in Form von Palladiumacetat auf ein Polystyren-4-Vinylpyridin-Blockcopolymer aufgebracht und anschließend reduziert wurde. In dem endgültigen Katalysator wurden die erhaltenen Palladium-Nanopartikel auf Aluminiumoxid aufgebracht.

DE-A 195 06 113 betrifft flüssige kolloidale Metallzubereitungen, die Micellen enthalten, die aus einem Blockcopolymerisat bestehen, das mindestens einen im Lösungsmittel solvatisierenden Polymerblock und einen für das kolloide Metall bindefähigen Polymerblock aufweist, in einem flüssigen organischen oder anorganischen Lösungsmittel. In keiner der genannten Veröffentlichungen - betreffend metallkolloidkatalysierte Reaktionen - wird die Verwendung von überkritischen Fluiden offenbart.

WO 01/14289 betrifft ein Verfahren zur Durchführung von katalytischen Reaktionen in überkritischem Kohlendioxid, worin eine Fluidmischung, die mindestens einen Reaktanden und Kohlendioxid enthält, mit einem Katalysator, der an ein Polymer gebunden ist, in Kontakt gebracht wird und der Reaktand mit dem Katalysator in Wechselwirkung tritt unter Ausbildung eines Reaktionsproduktes. Das eingesetzte Polymer kann entweder in Kohlendioxid löslich oder unlöslich sein. Als geeignete lösliche Polymere werden Polymere mit CO₂-philen Gruppen genannt. Als Beispiele für CO₂-phile Gruppen sind Silikon enthaltende Gruppen oder Polysiloxane, Halogen (insbesondere Fluor) enthaltende Gruppen oder Halogen- (insbesondere Fluoro-) Kohlenstoffe sowie verzweigte Polyalkylenoxide und fluorierte Polyether aufgeführt. Die Fluor enthaltende Einheit ist üblicherweise ein "Fluorpolymer". Als CO₂-phobe Gruppen werden in WO 01/14289 unter anderem aromatische Polymere sowie Oligomere genannt, die aus Styrol-, Acrylat- oder Vinylpyrrolidon-Monomeren aufgebaut sind.

Die im Stand der Technik bekannten Verfahren zur Wasserstoffübertragung weisen somit die folgenden Nachteile auf:
- Schlechte Abtrennbarkeit der Produkte.
- Schlechte Abtrennbarkeit des Katalysators.
- Niedrige Reaktionsgeschwindigkeiten.
- Die kationisch-homogene Katalyse im überkritischen Fluid ist wegen der geringen Löslichkeit der traditionellen Katalysatorsysteme nur mit fluorierten Liganden oder unter Zuhilfenahme fluorierter Cosolventien anwendbar.
- Die Katalysatoren für die kationisch-homogene Katalyse in überkritischen Systemen sind teuer und wegen des Einsatzes von Organofluorverbindungen umweltbedenklich.
- Nichtionisch-homogene Katalysatoren zeigen nur geringe Reaktivität.
- Die Ausnutzung des katalytisch aktiven Metalls bei heterogener Katalyse ist niedrig.
- Das Totvolumen durch Trägermaterial bei heterogener Katalyse ist störend.
- Produkte enthalten immer Spuren von Lösungsmitteln. Der technische Aufwand zur Produktreinigung, gerade im Bereich der Pharamzeutika und Lebensmittel ist hoch.
- Problem der Diffusionslimitierung bei Reaktionen mit Gasen in Zweiphasen-Systemen.
- Die Umsatzraten sind speziell in überkritischen Fluiden sehr niedrig.

Aufgabe der vorliegenden Erfindung ist es daher, ein Verfahren zur Wasserstoffiibertragung auf chemische Verbindungen bereitzustellen, das sich durch eine hohe Katalysatorausnutzung und kurze Reaktionszeiten, d.h. hohe Umsatzraten, auszeichnet. Des weiteren soll eine hohe Reaktivität des eingesetzten Katalysators bei milden Reaktionsbedingungen und geringer Nebenproduktbildung erzielt werden.

Diese Aufgabe wird durch ein Verfahren zur Wasserstoffübertragung auf chemische Verbindungen, wobei die Wasserstoffübertragung in überkritischer Phase unter Verwendung von stabilisierten und dispergierten Metallpartikeln durchgeführt wird. Bevorzugt werden die Metallpartikel in Form von Metallclustern oder Partikeln in kolloidaler Größenordnung eingesetzt.

Metallkolloide sind Systeme, in denen Metallteilchen mit einem Durchmesser in der Größenordnung von 1 nm bis 1 µm vorliegen. Das extrem feinteilige Metall selbst wird als kolloides Metall bezeichnet.

Unter Metallclustem werden Metallteilchen verstanden, die nur aus wenigen - einigen bis einigen Tausend - Metallatomen bestehen und am unteren Ende der Größenskala für kolloides Metall (Durchmesser der Metallteichen in Metallkolloiden: 1nm bis 1 µm) liegen.

Die vorliegende Erfindung betrifft ein Verfahren zur Wasserstoffübertragung auf chemische Verbindungen, worin die chemischen Verbindungen in Anwesenheit von dispergierten, durch Blockcopolymere stabilisierten oder in Blockcopolymere eingebetteten Metallkolloiden als Katalysatoren umgesetzt werden.

Das erfindungsgemäße Verfahren ist dann dadurch gekennzeichnet, dass die Umsetzung in überkritischer oder in subkritischer Phase durchgeführt wird.

Für die allgemein geläufigen Begriffe "überkritisch" und "subkritisch" (gleichbedeutend mit "nahekritisch") siehe z.B. den Artikel "Interaction of Density, Viscosity and Interfacial Tension in Countercurrent Extraction with Near-Critical Fluids" in "High Pressure Chemical Engineering", Seiten 191 bis 197, Ed. Ph. Rudolf von Rohr und Ch. Trepp, Elsevier Science B.V. 1996 und die darin zitierte Literatur.
Das erfindungsgemäße Verfahren zeichnet sich durch hohe Reaktionsgeschwindigkeiten und somit eine hohe Effizienz aus. Aufgrund des Einsatzes von überkritischem Lösungsmittel bleiben keine gegebenenfalls toxischen Lösungsmittelrückstände im Produkt zurück, und Katalysator und Produkt lassen sich leicht abtrennen. Die gasförmigen Reaktanden zeigen eine maximale Aktivität. Mit Hilfe des erfindungsgemäßen Verfahrens lassen sich die Vorteile der heterogenen Katalyse (z.B. gute Abtrennbarkeit) und der homogenen Katalyse (z.B. Katalysatorausnutzung, Selektivität) kombinieren.

Somit ergeben sich für das erfindungsgemäße Verfahren folgende Vorteile:
- Keine toxischen Lösungsmittelrückstände im Produkt.
- Hohe Effizienz.
- Leichte Abtrennbarkeit von Katalysator und Produkt.
- Gefundene Reaktionsgeschwindigkeiten übertreffen alle bisher bekannten.
- Preisgünstige, technisch zugängliche Katalysatoren.
- Verminderte Umweltbelastung.
- Niedriger Energieverbrauch durch milde Reaktionsbedingungen.
- Maximale Aktivität gasförmiger Reaktanden.
- Vorteile der heterogenen Katalyse (gute Abtrennbarkeit etc.) und der homogenen Katalyse (Katalysatorausnutzung, Selektivität etc.) lassen sich in überkritischen Systemen kombinieren.

Durch den erfindungsgemäßen Einsatz von überkritischen Lösungsmitteln ist die Wasserstoffkonzentration in dem Lösungsmittel nahezu frei wählbar und nicht mehr ein beschränkender Faktor bei der Reaktion wie in flüssigen Lösungsmitteln, worin die Löslichkeit von Wasserstoff gering ist. Das Wort überkritisch bezieht sich auf den Zustand des eingesetzten Lösungsmittels (Fluids). Beispielsweise liegt Kohlendioxid in überkritischem Zustand vor, wenn die Temperatur über 31,1° C liegt und gleichzeitig der Druck über 73,8 bar liegt. Überkritische Fluide zeigen sowohl flüssigkeitsartige als auch gasartige Eigenschaften, z.B. eine flüssigkeitsartige Dichte und eine gasartige Viskosität. Das Diffusionsvermögen von überkritischen Fluiden liegt zwischen den Werten von Gasen und Flüssigkeiten. Aufgrund eines verstärkten Stofftransports sind gasartige Eigenschaften in der Reaktionschemie vorteilhaft.

Eine besonders wichtige Eigenschaft von überkritischen Fluiden besteht darin, dass sie mit jeder Art von Gasen, einschließlich Wasserstoffgas, vollständig mischbar sind. Das heißt, dass, wenn das Lösungsmittel in überkritischem Zustand ist, das zur Reduktion eingesetzte Wasserstoffgas in einfacher Weise mit dem Lösungsmittel gemischt werden kann.

Bei der Durchführung des erfindungsgemäßen Verfahrens hängt die Auswahl des geeigneten Lösungsmittels von der Art der Wasserstoffübertragungsreaktion sowie den eingesetzten Ausgangsprodukten ab. Geeignet sind insbesondere aromatische und aliphatische Kohlenwasserstoffe, wie Benzol, Toluol, Ethan, Propan oder Butan; Kohlendioxid; Alkohole, wie Methanol oder Ethanol; oder Mischungen davon. Bevorzugt werden Kohlendioxid sowie niedermolekulare Kohlenwasserstoffe, wie Ethan oder Propan eingesetzt. Ganz besonders bevorzugt enthält die überkritische oder subkritische Phase Kohlendioxid. Kohlendioxid ist umweltverträglich, ungiftig, nicht entflammbar, preiswert, korrosionsbeständig und ohne weiteres erhältlich. Es kann in dem erfindungsgemäßen Verfahren zur Wasserstoffübertragung gleichzeitig als Lösungsmittel und Schutzgas fungieren.

Es ist möglich, das Verhalten von überkritischen Lösungsmitteln durch Zugabe eines Modifiziermittels, z.B. kurzkettige Alkohole oder Ester, zu modifizieren.

Die Herstellung von Metallkolloiden ist seit langem bekannt. Üblicherweise werden Metallsalze in Lösung in Gegenwart von Stabilisatoren zum Metall reduziert (siehe z.B. G. Schmid, VCH-Verlag 1994, Clusters and Colloids). Bei den Stabilisatoren handelt es sich um Substanzen, die in der Lage sind, koordinative Bindungen zum Metall einzugehen, und es dadurch vor Agglomeration zu schützen. In dem Verfahren gemäß der vorliegenden Anmeldung werden als Stabilisatoren Blockcopolymere eingesetzt.

Die durch Blockcopolymere stabilisierten oder in Blockcopolymere eingebetteten Metallkolloide werden im folgenden als stabilisierte Metallkolloide bezeichnet. Bevorzugt handelt es sich bei den stabilisierten Metallkolloiden um in Blockcopolymere eingebettete Metallkolloide.

Die stabilisierten Metallkolloide sind bevorzugt in der überkritischen Phase dispergierbar. Es ist bekannt, dass die Löslichkeit von Polymeren in einer überkritischen Phase, insbesondere in überkritischem Kohlendioxid, mäßig bis schlecht ist. Gemäß WO 01/14289 sind Polymere, die CO₂-phile Gruppen enthalten, in Kohlendioxid löslich. Als CO₂-phile Gruppen werden Silikon enthaltende Gruppen oder Polysiloxane, Halogen (insbesondere Fluor) enthaltende Gruppen oder Halogen- (insbesondere Fluoro-) Kohlenstoffe sowie verzweigte Polyalkylenoxide und fluorierte Polyether genannt. Es wurde überraschenderweise gefunden, dass durch den Einsatz von Blockcopolymeren als Stabilisatoren, bevorzugt von Blockcopolymeren, worin mindestens zwei Polymerblöcke ausgewählt aus der Gruppe bestehend aus Polystyren, Polyalkylstyren, Polyisopren, Polymethyl(meth)acrylat und Polybutadien, Poly-4-vinylpyridin, Poly-2-vinylpyridin, Polyethylenglycol, Polyethylenoxid, Poly(meth)acrylsäure, Polyhydroxy-ethylmethacrylat, Polyvinylalkohol, Polydimethylsiloxan, Poly-2-dimethylamino-ethyl(meth)acrylat und Polyethylethylen eingesetzt werden, Katalysatoren erhalten werden, die in der überkritischen Phase, insbesondere in überkritischem Kohlendioxid, in kolloider Form dispergiert vorliegen. Besonders bevorzugt werden als Stabilisatoren Blockcopolymere ausgewählt aus Polystyren-poly-4-vinylpyridin, Polystyren-poly-2-vinylpyridin, Polystyren-poly-(meth)acrylsäure, Polystyren-poly-ethylenglycol, Polystyren-poly-ethylenoxid, Polystyren-poly-hydroxy-ethylmethacrylat, Polystyren-poly-vinylalkohol, Polydimethylsiloxan-poly-ethylenoxid, Poly-2-dimethylamino-ethylmethacrylat-polymethylmethacrylat und Polyethylenoxid-polyethylethylen eingesetzt. Dabei kann anstelle von Polystyrol aus Polymethylmethacrylat eingesetzt werden. Ganz besonders bevorzugt werden Polystyren-poly-4-vinylpyridin-Blockcopolymere als Stabilisatoren eingesetzt. Der Einsatz von in WO 01/14289 genannten Polymeren, die aufgrund der in WO 01/14289 aufgeführten CO₂-philen Gruppen teuer sind, ist nicht erforderlich.

Die Herstellung der erfindungsgemäß eingesetzten Blockcopolymere ist dem Fachmann bekannt. Sie können z.B. durch anionische Polymerisation der entsprechenden Monomere erhalten werden (M. Antonietti, Chem. Mater. 1997, Nr. 9, 923-931). Die Herstellung der besonders bevorzugt eingesetzten Polystyren-Poly-4-vinylpyridin-Blockcopolymere erfolgt nach einer in Bronstein et al., J. Catal. 196, 302 bis 312 (2000) offenbarten Vorschrift.

Die Metallkolloide sind in einer bevorzugten Ausführungsform in die genannten Blockcopolymere eingebettet. Solche stabilisierten Metallkolloide werden z.B. gemäß M. Antonietti, Chem. Mater. 1997, Nr. 9, 923-931 erhalten.

Bei den Metallkolloiden handelt es sich im Sinne der vorliegenden Anmeldung um Metalle, Metallegierungen oder nicht legierte Metallkombinationen. Diese sind bevorzugt ausgewählt aus einem oder mehreren Metallen aus der Gruppe bestehend aus Nickel, Cobalt, Palladium, Platin, Gold, Silber, Kupfer, Rhodium, Ruthenium und Iridium, besonders bevorzugt werden ein oder mehrere Metalle ausgewählt aus der Gruppe bestehend aus Palladium, Gold, Platin, Rhodium und Ruthenium eingesetzt.

Ganz besonders bevorzugt weisen die erfindungsgemäßen Metallkolloide einen Kern, ausgewählt aus einem der vorstehend genannten Metalle, insbesondere aus Gold auf, der mit einer Schicht aus einem weiteren, von dem Metall des Kerns verschiedenen Metall ausgewählt aus den vorstehend genannten Metallen, bevorzugt Palladium, umgeben ist. Zur Herstellung der Metallkolloide, aufgebaut aus einem Kern und einer Schale siehe z.B. G. Schmid, VCH-Verlag 1994, Clusters and Colloids und Bronstein et al., J. Catal. 2000, Vol. 196, S. 302-314.

Zur Stabilisierung und Dispergierung der Metallpartikel kann gegebenenfalls ein Cosolvens eingesetzt werden. Bevorzugt ist ein Cosolvens ausgewählt aus der Gruppe bestehend aus Toluol, o-, m-, p-Xylol, α,α,α- Trifluorotoluol, Benzol, Ethylbenzol, Cyclohexan, Hexan, Pentan und teilfluorierten Alkoholen.

Bei der Wasserstoffübertragungsreaktion handelt es sich bevorzugt um eine Reaktion ausgewählt aus der Gruppe bestehend aus Hydrierung, Hydroformylierung, Hydrogenolyse, Hydrocarboxylierung und Hydrosilylierung. Besonders bevorzugt wird eine Hydrierung oder Hydroformylierung, ganz besonders bevorzugt eine Hydrierung durchgeführt.

Die Reägenzien, mit denen die chemischen Verbindungen in Anwesenheit von stabilisierten Metallkolloiden als Katalysatoren in überkritischer Phase umgesetzt werden, sind abhängig von der jeweiligen Wasserstoffübertragungsreaktion und entsprechen den in den entsprechenden aus dem Stand der Technik bekannten Verfahren eingesetzten Reagenzien.

Die chemischen Verbindungen, die in dem erfindungsgemäßen Verfahren umgesetzt werden sind ebenfalls abhängig von der jeweiligen Wasserstoffübertragungsreaktion und entsprechen den in den entsprechenden aus dem Stand der Technik bekannten Verfahren eingesetzten chemischen Verbindungen.

Ist die Wasserstoffübertragungsreaktion eine bevorzugt durchgeführte Hydroformylierung, so werden Wasserstoff und Kohlenmonoxid als Reagenzien eingesetzt. Das erfindungsgemäße Verfahren kann auf alle gängigen Hydroformylierungsreaktionen an chemischen Verbindungen angewendet werden. Die in der gemäß dem erfindungsgemäßen Verfahren durchgeführten Hydroformylierung eingesetzten chemischen Verbindungen sind bevorzugt C₂- bis C₂₀-Olefine oder Alkine.

In der besonders bevorzugten Hydrierungsreaktion erfolgt eine Umsetzung der chemischen Verbindungen mit Wasserstoff als Reagenz. Das erfindungsgemäße Verfahren kann auf alle gängigen Hydrierungsreaktionen an chemischen Verbindungen angewendet werden. Bevorzugt in der gemäß dem erfindungsgemäßen Verfahren durchgeführten Hydrierung eingesetzte chemische Verbindungen sind ausgewählt aus der Gruppe bestehend aus Alkinen; Alkinolen; Alkenen, insbesondere ungesättigten Fettsäuren; Nitroverbindungen; Carbonsäuren, insbesondere Fettsäuren; Carbonylverbindungen und aromatischen Verbindungen.

Besonders bevorzugte chemische Verbindungen sind Alkine und Alkinole, ganz besonders bevorzugt 1-Hexin, 3-Methylpent-1-in-3-ol, 3-Phenylpropin, 3,3-Dimethylbutin und Phenylethin.

Die mit dem erfindungsgemäßen Verfahren durchführbaren Hydrierungsreaktionen sind unselektive (d.h. vollständige) und selektive Hydrierungen. Ein Beispiel für eine selektive Hydrierung ist die Hydrierung von Alkinen zu Alkenen.

Grundsätzlich kann das Verfahren bei beliebigen Druck/Temperatur-Kombinationen durchgeführt werden, solange gewährleistet ist, dass eine überkritische Phase vorliegt. Die genauen Druck- und Temperaturwerte sind von der entsprechenden Wasserstoffübertragungsreaktion und dem eingesetzten Lösungsmittel abhängig.

Wird das Verfahren in Kohlendioxid durchgeführt, so wird es im allgemeinen bei Temperaturen von 32 bis 250°C, bevorzugt von 40 bis 100 °C, besonders bevorzugt von 50 °C und Drucken von 74 bis 350 bar, bevorzugt von 100 bis 200 bar, besonders bevorzugt von 150 bar Kohlendioxiddruck (zuzüglich des Drucks, der durch den Partialdruck des gegebenenfalls als Reagenz eingesetzten Gases verursacht wird (z.B. Hydrierung: H₂; Hydroformylienmg: CO und H₂) durchgeführt. Die überkritische Phase wird üblicherweise dadurch erzeugt, dass das als Lösungsmittel eingesetzte Fluid, bevorzugt Kohlendioxid, in einem Reaktor bei der genannten Temperatur auf den genannten Druck verdichtet wird. Die Zugabe der chemischen Verbindungen, des Katalysators und des in Abhängigkeit von der Wasserstoffübertragungsreaktion eingesetzten Reagenzes, sowie gegebenenfalls weiterer Komponenten, kann vor oder nach der Verdichtung erfolgen. Bevorzugt erfolgt zunächst die Zugabe der chemischen Verbindung, des Katalysators, des in Abhängigkeit von der Wasserstoffübertragungsreaktion eingesetzten Reagenzes, sowie gegebenenfalls weiterer Komponenten zu dem als Lösungsmittel eingesetzten Fluid und anschließend die Verdichtung auf die genannte Temperatur und den genannten Druck zur Erzeugung der überkritischen oder subkritischen Phase.

Wird eine besonders bevorzugte Hydrierung durchgeführt, wird bevorzugt soviel Wasserstoff zugegeben, dass ein Wasserstoffpartialdruck von im allgemeinen 1 bis 100 bar, bevorzugt von 2 bis 50 bar, besonders bevorzugt von 2 bis 15 bar (Druckerhöhung bezogen auf den vorher eingestellten Reaktorinnendruck) eingestellt wird.

Die eingesetzte Menge des als Katalysator eingesetzten stabilisierten Metallkolloids ist abhängig von der durchgeführten Wasserstoffübertragungsreaktion. In der besonders bevorzugten Hydrierung beträgt die Menge im allgemeinen 0,01 bis 5 Gew.-% bevorzugt 0,02 bis 2 Gew.-%, besonders bevorzugt 0,1 bis 1 Gew.-%, bezogen auf die eingesetzte chemische Verbindung.

Die Reaktionsdauer ist ebenfalls abhängig von der durchgeführten Wasserstoffübertragungsreaktion, sowie von den eingesetzten chemischen Verbindungen. Üblich ist eine Reaktionsdauer von 1 Minute bis 1 Stunde, bevorzugt 1 Minute bis 10 Minuten.

Die mit dem erfindungsgemäßen Verfahren erzielten Umsatzraten sind wesentlich höher als die in flüssiger Phase erzielten Umsätze. Für die besonders bevorzugt durchgeführte Hydrierung werden Umsatzraten von im allgemeinen 30000 h⁻¹ bis 8000000 h⁻¹, bevorzugt 100000 h⁻¹ bis 4000000 h⁻¹, besonders bevorzugt 500000 h⁻¹ bis 2000000 h⁻¹ erzielt.

Das erfindungsgemäße Verfahren kann kontinuierlich, semi-kontinuierlich oder im Batch-Verfahren durchgeführt werden, wobei eine kontinuierliche Fahrweise bevorzugt ist.

Die als Katalysatoren erfindungsgemäß eingesetzten stabilisierten Metallkolloide können nach der Umsetzung zurückgewonnen und wieder eingesetzt werden. Nicht umgesetzte chemische Verbindungen können ebenfalls zurückgewonnen und wieder eingesetzt werden.

In einer bevorzugten Ausführungsform des erfindungsgemäßen Verfahrens wird zur Erzeugung einer überkritischen Phase das Lösungsmittel verdichtet, so dass es in einer überkritischen oder subkritischen Phase vorliegt. Anschließend wird - bei einer besonders bevorzugten Hydrierung - Wasserstoff zugegeben. Zu dieser Mischung wird die zu hydrierende chemische Verbindung sowie das erfindungsgemäß als Katalysator eingesetzte stabilisierte Metallkolloid gegeben. Im Anschluß an die Umsetzung können die Komponenten z.B. durch Filtration, Ultrafiltration, Destillation oder Sedimentation getrennt werden.

Für das erfindungsgemäße Verfahren sind alle üblicherweise für Umsetzungen in überkritischer oder subkritischer Phase eingesetzten Reaktoren wie Rührkessel oder Blasensäulen geeignet.

Durch Variation der Reaktionsbedingungen, der eingesetzten Metalle, Metalllegierungen oder nicht legierten Metallkombinationen, der zur Stabilisierung der Metallkolloide eingesetzten Stabilisatoren, gegebenenfalls durch Zugabe weiterer Substanzen in Abhängigkeit von der jeweiligen Wasserstoffübertragungsreaktion, gegebenenfalls durch vorgelagerte Reaktionen oder durch physikalische Effekte, können die Reaktivität und Selektivität der Produktbildung gesteuert werden, wobei die Variation bevorzugt in den vorstehend genannten Bereichen erfolgt.

Das erfindungsgemäße Verfahren zeichnet sich insbesondere dadurch aus, dass durch den Einsatz von stabilisierten Metallkolloiden als Katalysatoren in dem erfindungsgemäßen Verfahren eine simultane, d.h. paarweise, Übertragung der beiden Wasserstoffatome eines Wasserstoffmoleküls erfolgt, was für eine homogene Hydrierung typisch ist. Durch die paarweise Übertragung der Wasserstoffatome werden radikalische Zwischenstufen bei der Wasserstoffübertragung vermieden. Durch die Vermeidung der radikalischen Zwischenstufen wird die Bildung von Nebenprodukten, die bei der Bildung radikalischer Zwischenstufen aus diesen entstehen können, vermieden.

Ein weiterer Gegenstand der vorliegenden Anmeldung ist daher die Verwendung von durch organische Verbindungen stabilisierten oder in organische Verbindungen eingebetteten Metallkolloiden als Katalysatoren in einem Verfahren zur Wasserstoffübertragung auf chemische Verbindungen in überkritischer oder in subkritischer Phase zur Vermeidung der Bildung radikalischer Zwischenstufen während der Wasserstoffübertragung.

Dadurch wird die Bildung von Nebenprodukten aufgrund von Nebenreaktionen üblicherweise gebildeter radikalischer Zwischenstufen vermindert.

Geeignete organische Verbindungen, Metallkolloide, Verfahren zur Wasserstoffübertragung und überkritische Lösungsmittel wurden bereits vorstehend genannt.

Das nachfolgende Beispiel beschreibt die Erfindung zusätzlich:

### Beispiel

In einem geeigneten Reaktor bzw. Autoklaven, in diesem Falle ein NMR-Hochdruckprobenkopf, werden Substrate und Katalysator vorgelegt. Als Katalysator dienen gemischte Übergangsmetallkolloide, welche aus Goldkernen bestehen, die von einer Palladiumschicht umgeben sind. Die hier stellvertretend verwendeten Kolloide sind stabilisiert durch Polystyren-poly-4-vinylpyridin-Blockcopolymere (Bronstein et al. J. Catal. 196, 302-312 (2000)). Als charakteristische Substrate werden wahlweise 1-Hexin, 3-Methylpent-1-in-3-ol, 3-Phenylpropin, 3,3-Dimethylbutin und Phenylethin eingesetzt. Zur Erzeugung einer überkritischen Lösung wird im Reaktor Kohlenstoffdioxid bei einer Temperatur von 50 °C auf einen Druck von 150 bar verdichtet. Anschließend wird mit einer Kolbenpumpe (ISCO 100 DM, Lincoln, Nebraska, USA) Wasserstoff in den Reaktor gegeben. In getrennten Versuchen wird jedes der angegebenen Substrate bei einem konstanten Wasserstoffüberdruck (15 bar Druckerhöhung bezogen auf den vorher eingestellten Reaktorinnendruck von 150 bar CO₂) hydriert. Der Verlauf der jeweiligen Reaktion wird *in situ* durch NMR-Messungen verfolgt. Durch Auswertung der sich im Verlauf der Reaktion ändernden Signale, z.B. des Alkinwasserstoffsignals, lässt sich der Reaktionsfortschritt der jeweiligen Reaktion ermitteln. Bei allen eingesetzten Substraten sind die ermittelten Umsatzraten wesentlich höher als die, die in organischen Lösungsmitteln erhalten werden. Zur Ermittlung der Druckabhängigkeit der untersuchten Reaktion werden sowohl von Phenylethin als auch 3-Methyl-pent-1-in-3-ol zusätzlich Hydrierungen bei 2, 4, 8, 10 und 15 bar durchgeführt. Über den genannten Druckbereich (d.h. von 2-15 bar Wasserstoffüberdruck) wird eine Steigerung der Umsatzraten mit Erhöhung des Wasserstoffdruckes um einen Faktor von 15 für Phenylethin und einen Faktor von 20 für 3-Methylpent-1-in-3-ol beobachtet.

Bei einem CO₂-Druck von 150 bar, einem H₂-Druck (aufgepresst auf die vorgelegte Menge CO₂) von 15 bar und einem Substrat-Katalysator-Verhältnis von ca. 6500 und einer Temperatur von 50 °C ergeben sich die folgenden Umsätze (turnover frequencies (TOF)):

| | |
|---|---|
| 1-Hexin | 1180000 h⁻¹ |
| Phenylethin | 500400 h⁻¹ |
| 3-Hydroxy-3-methyl-1-pentin | 432100 h⁻¹ |
| 1-Phenyl-3-propin | 236200 h⁻¹ |
| 3,3-Dimethyl-1-butin | 104700 h⁻¹ |

In Hexan ergeben sich demgegenüber maximale Umsätze von etwa 30000 h⁻¹.

## Patentansprüche

1. Verfahren zur Wasserstoffübertragung auf chemische Verbindungen, worin die chemischen Verbindungen in Anwesenheit von dispergierten, durch Blockcopolymere stabilisierten oder in Blockcopolymere eingebetteten Metallkolloiden als Katalysatoren umgesetzt werden, **dadurch gekennzeichnet, dass** die Umsetzung in überkritischer Phase durchgeführt wird.

2. Verfahren nach Anspruch 1, **dadurch gekennzeichnet, dass** die Blockcopolymere ausgewählt sind aus Polystyren-poly-4-vinylpyridin, Polystyren-poly-2-vinylpyridin, Polystyren-poly-(meth)-acrylsäure, Polystyren-poly-ethylenglycol, Polystyren-poly-ethylenoxid, Polystyren-poly-hydroxy-ethylmethacrylat, Polystyren-poly-vinylalkohol, Polydimethylsiloxan-poly-ethylenoxid, Poly-2-dimethylamino-ethylmethacrylat-polymethylmethacrylat und Polyethylenoxid-polyethylethylen.

3. Verfahren nach Anspruch 2, **dadurch gekennzeichnet, dass** als Blockcopolymere Polystyren-poly-4-vinylpyridin-Blockcopolymere eingesetzt werden.

4. Verfahren nach einem der Ansprüche 1 bis 3, **dadurch gekennzeichnet, dass** die Metallkolloide Metalle, Metalllegierungen oder nicht legierte Metallkombinationen ausgewählt aus einem oder mehreren Metallen aus der Gruppe bestehend aus Nickel, Cobalt, Palladium, Platin, Gold, Silber, Kupfer, Rhodium, Ruthenium und Iridium enthalten.

5. Verfahren nach Anspruch 4, **dadurch gekennzeichnet, dass** die Metallkolloide einen Goldkern aufweisen, der von einer Palladiumschicht umgeben ist.

6. Verfahren nach einem der Ansprüche 1 bis 5, **dadurch gekennzeichnet, dass** die überkritische Phase überkritisches Kohlendioxid enthält.

7. Verfahren nach einem der Ansprüche 1 bis 6, **dadurch gekennzeichnet, dass** die Wasserstoffübertragung eine Reaktion ausgewählt aus der Gruppe bestehend aus Hydrierung, Hydroformylierung, Hydrogenolyse, Hydrocarboxylierung und Hydrosilylierung ist.

8. Verfahren nach Anspruch 7, **dadurch gekennzeichnet, dass** die Wasserstoffübertragung eine Hydrierung ist.

9. Verfahren nach Anspruch 8, **dadurch gekennzeichnet, dass** die Hydrierung bei einem Wasserstoffpartialdruck von 2 bis 15 bar durchgeführt wird.

10. Verwendung von durch Blockcopolymere stabilisierten oder in Blockcopolymere eingebetteten Metallkolloiden als Katalysatoren in einem Verfahren zur Wasserstoffübertragung auf chemische Verbindungen in überkritischer Phase zur Vermeidung der Bildung radikalischer Zwischenstufen während der Wasserstoffübertragung.

## Claims

1. A process for hydrogen transfer to chemical compounds in which the chemical compounds are reacted in the presence of catalysts comprising dispersed metal colloids stabilized by block copolymers or embedded in block copolymers, wherein the reaction is carried out in a supercritical phase.

2. The process according to claim 1, wherein the block copolymers are selected from the group consisting of polystyrene-poly-4-vinylpyridine, polystyrene-poly-2-vinylpyridine, polystyrene-poly(meth)acrylic acid, polystyrene-polyethylene glycol, polystyrene-polyethylene oxide, polystyrene-polyhydroxyethyl methacrylate, polystyrene-polyvinyl alcohol, polydimethylsiloxane-polyethylene oxide, poly-2-dimethylaminoethyl methacrylate-polymethyl methacrylate and polyethylene oxide-polyethylethylene.

3. The process according to claim 2, wherein the block copolymers used are polystyrene-poly-4-vinylpyridine block copolymers.

4. The process according to any of claims 1 to 3, wherein the metal colloids comprise metals, metal alloys or unalloyed metal combinations consisting of one or more metals selected from the group consisting of nickel, cobalt, palladium, platinum, gold, silver, copper, rhodium, ruthenium and iridium.

5. The process according to claim 4, wherein the metal colloids comprise a gold core surrounded by a layer of palladium.

6. The process according to any of claims 1 to 5, wherein the supercritical phase comprises supercritical carbon dioxide.

7. The process according to any of claims 1 to 6, wherein the hydrogen transfer is a reaction selected from the group consisting of hydrogenation, hydroformylation, hydrogenolysis, hydrocarboxylation and hydrosilylation.

8. The process according to claim 7, wherein the hydrogen transfer is a hydrogenation.

9. The process according to claim 8, wherein the hydrogenation is carried out at a hydrogen partial pressure of from 2 to 15 bar.

10. The use of metal colloids stabilized by block copolymers or embedded in block copolymers as catalysts in a process for hydrogen transfer to chemical compounds in a supercritical phase so as to avoid the formation of free-radical intermediates during hydrogen transfer.

## Revendications

1. Procédé de transport d'hydrogène sur des composés chimiques, dans lequel les composés chimiques sont mis à réagir en présence de colloïdes métalliques dispersés, stabilisés par des copolymères blocs ou noyés dans des copolymères blocs, en tant que catalyseurs, **caractérisé en ce que** la réaction est effectuée en phase surcritique.

2. Procédé suivant la revendication 1, **caractérisé en ce que** les copolymères blocs sont choisis parmi de la polystyrène-poly-4-vinylpyridine, de la polystyrène-poly-2-vinylpyridine, de l'acide polystyrène-poly-(méth)acrylique, du polystyrène-poly-éthylèneglycol, de l'oxyde de polystyrène-poly-éthylène, du méthacrylate de polystyrène-poly-hydroxy-éthyle, de l'alcool polystyrène-poly-vinylique, de l'oxyde de polydiméthylsiloxane-polyéthylène, du méthacrylate de poly-2-diméthylamino-éthyle-polyméthacrylate de méthyle et de l'oxyde de polyéthylène-polyéthyléthylène.

3. Procédé suivant la revendication 2, **caractérisé en ce que**, comme copolymères blocs, on met en oeuvre des copolymères blocs de polystyrène-poly-4-vinylpyridine.

4. Procédé suivant l'une des revendications 1 à 3, **caractérisé en ce que** les colloïdes métalliques contiennent des métaux, des alliages métalliques ou des combinaisons métalliques non alliées choisis parmi un ou plusieurs métaux du groupe constitué de nickel, de cobalt, de palladium, de platine, d'or, d'argent, de cuivre, de rhodium, de ruthénium et d'iridium.

5. Procédé suivant la revendication 4, **caractérisé en ce que** les colloïdes métalliques présentent un noyau d'or qui est entouré d'une couche de palladium.

6. Procédé suivant l'une des revendications 1 à 5, **caractérisé en ce que** la phase surcritique contient du dioxyde de carbone surcritique.

7. Procédé suivant l'une des revendications 1 à 6, **caractérisé en ce que** le transport d'hydrogène est une réaction choisie parmi le groupe constitué d'une hydrogénation, d'une hydroformylation, d'une hydrogénolyse, d'une hydrocarboxylation et d'une hydrosilylation.

8. Procédé suivant la revendication 7, **caractérisé en ce que** le transport d'hydrogène est une hydrogénation.

9. Procédé suivant la revendication 8, **caractérisé en ce que** l'hydrogénation est effectuée à une pression partielle d'hydrogène de 2 à 15 bars.

10. Utilisation de colloïdes métalliques stabilisés par des copolymères blocs ou noyés dans des copolymères blocs, comme catalyseurs dans un procédé pour le transport d'hydrogène sur des composés chimiques en phase surcritique, en vue d'éviter la formation d'étapes intermédiaires radicalaires pendant le transport d'hydrogène.
